# EUROPEAN PATENT APPLICATION

(11) **EP 2 388 032 A1**
(43) Date of publication of application: **23.11.2011**
(21) Application number: 10163670.2
(22) Date of filing: 21.05.2010
(51) Int. Cl.: A61M 5/30, A61B 17/3203

(54) **Device for creating a microfluidic jet and uses thereof**

(71) Applicant: UNIVERSITEIT TWENTE, 7522 NB Enschede (NL)
(72) Inventor: Prosperetti, Andrea, 7500 AE Enschede (NL); Lohse, Detlef, 7500 AE Enschede (NL); Dijkink, Rory Jan, 7511 PV Enschede (NL); Sun, Chao, 7500 AE Enschede (NL)
(74) Representative: Holzwarth-Rochford, Andreas

(57) **Abstract**

A device for creating at least one microfluidic jet, includes at least one conduit section (4a-c;14;22;27), open to an environment of the device at one end thereof. It further includes at least one arrangement (18) for forming in at least one of the conduit sections (4a-c;14;22;27) a meniscus (24;34) forming an interface between liquid (23) in the conduit section (4a-c;14;22;27) and the environment, at a position within the conduit section (4a-c;14;22;27). The meniscus (24;34) is at least partially concave, seen looking into the conduit section (4a-c;14;22;27) through the end open to the environment. The device includes at least one holder (13) for holding liquid (23), in fluid communication with at least one of the conduit sections (4a-c;14;22;27). It also includes at least one device (8;11;28) for delivering an energy pulse to liquid (23) in at least one of the holders (13), configured to create a shock front propagating to the meniscus (24;34) and causing at least a first stage (25;33) of a jet to emerge from a central part of the meniscus (24;34).

## Description

The invention relates to a device for creating at least one microfluidic jet, including:
at least one conduit section, open to an environment of the device at one end thereof;
at least one arrangement for forming in at least one of the conduit sections a meniscus forming an interface between liquid in the conduit section and the environment, at a position within the conduit section,
wherein the meniscus is at least partially concave, seen looking into the conduit section through the end open to the environment; and
at least one holder for holding liquid, in fluid communication with at least one of the conduit sections.

The invention also relates to a method of creating at least one microfluidic jet, including:
providing at least one conduit section, open to an environment at one end; and
providing liquid within at least one of the conduit sections up to a meniscus forming an interface between liquid in the conduit section and the environment,
wherein the meniscus is situated at a position within the conduit section, and
wherein the meniscus is at least partly concave, seen looking into the conduit section through the end open to the environment.

The invention also relates to a device for providing an injection to an organism.

The invention also relates to uses of a method of creating a jet and/or a device for creating a jet.

US 2004/0260234 A1 discloses a repetitive microjet device comprising a drug reservoir in fluid communication with a microjet. The microjet generally includes a force generating mechanism, a chamber, and a nozzle. In use, the force generating mechanism generally functions to change the pressure within the chamber, thereby accelerating injectate within the chamber toward the nozzle. According to an embodiment, the force generating mechanism is a piezoelectric mechanism. According to yet another embodiment, the force generation mechanism can be an electromagnetic actuation mechanism. According to yet another embodiment, the force generation mechanism can be a spring mechanism. In still a further embodiment, the force generation mechanism can be a highly pressurised gas which, when activated, moves a plunger and thereby displaces injectate from the nozzle. In still a further embodiment, the force generation mechanism can be an explosive mechanism. According to an alternative embodiment, the force generating mechanism can be a phase change mechanism. The phase change mechanism includes two electrodes. The chamber is a fully enclosed chamber that houses actuation fluid. A flexible membrane is preferably non-permeable to actuation fluid in the chamber and injectate contained in the nozzle, such that the two compositions do not mix. The actuation fluid is a fluid that is easily broken down and vaporises rapidly upon the build-up of a difference in electric charge on the electrodes. According to an alternative embodiment, the actuation fluid can be the injectate. Accordingly, the flexible membrane may not be necessary as the entire chamber and nozzle are filled with a fluid that is ultimately injected following activation of the phase change mechanism. In an alternative embodiment, actuation fluid can be maintained separate from the injectate by chemical and physical properties of the actuation fluid. Therefore, no membrane is required.

According to an embodiment, as shown in Fig. 19, the proximal end of the nozzle can include a coating to make it repel injectate, constructed from a composition that repels injectate. In this embodiment, the injectate is retained a set distance from the surface of a biological barrier during resting stages of the device. Therefore, if injectate has a tendency to irritate the biological barrier or produce another negative effect on the biological barrier if left in contact with the biological barrier, these events will be minimised. Furthermore, in accordance with this embodiment, a more accurate quantity of administered injectate can be predicted and delivered, because the injectate will not be able to diffuse through the biological barrier or enter the biological barrier except as the jet propulsion stream during administration.

A problem of the known method is that the jet diameter is determined by the diameter of the orifice of the nozzle. This puts a lower limit on the achievable jet diameter.

It is an object of the present invention to provide devices, a method and uses, with which a thin jet can be provided without having to force the fluid through a very narrow nozzle.

This object is achieved by the device according to the invention, which is characterised by

at least one device for delivering an energy pulse to liquid in at least one of the holders, configured to create a pressure pulse, in particular a shock front, propagating to the meniscus and causing at least a first stage of a jet to emerge from a central part of the meniscus.

The holder for holding liquid is a component part of the overall device that can be a further section of the same conduit as that comprising the conduit section in which the meniscus is formed. It need not be closed at its end opposite to where it joins the conduit section, but there is an unobstructed passage for liquid into the conduit section. It is observed that the term "microfluidic" is used herein to denote jets with at least a first stage having sub-millimetre dimensions, i.e. a diameter of 1 mm or less.

By creating a meniscus forming an interface between liquid in the conduit section and the environment, with the meniscus being at least partially concave, seen looking into the conduit section through the end open to the environment, it is possible to create a jet of which at least a first stage emerges from only a central part of the meniscus, but not the part closest to the wall of the conduit section. This is achieved by creating a shock front that passes over the meniscus. Surface corrugations due to the at least partly concave shape cause a velocity field that is directed away from the opening into the conduit section at the edges of the meniscus and in the opposite direction at the central part of the meniscus. This is due also to the fact that the meniscus is located at a position within the conduit section. Because an energy pulse is delivered, rather than a constant lower flow of energy, a pressure jump is created, that propagates to the meniscus. In particular in the case of needleless injection, at least the first stage of the jet of liquid has a relatively high velocity and small diameter, enabling it to penetrate the skin of an animal, in particular a human being, without causing bruising. In addition, the volume of the injected liquid can be very small and precisely contained. In use, an energy pulse with sufficient energy and of a sufficiently short duration that a steep pressure pulse propagating as a shock front to the meniscus and thereby causing at least a first stage of a jet to emerge from a central part of the meniscus is delivered. Because the energy pulse is delivered to liquid in a holder in unimpeded fluid communication with the conduit section, there are no membranes or other obstacles that prevent the propagation of the shock to the meniscus.

In an embodiment, the at least one device for delivering an energy pulse is configured to create the pressure pulse, in particular a shock front, by creating a vapour bubble in liquid in the at least one holder.

Because the device includes at least one device for delivering an energy pulse creating a vapour bubble in liquid in at least one holder in fluid communication with the conduit section, the shock is created in a practical way, enabling applications such as: needleless injection; localised cooling of device components, in particular electrical device components; micromachining workpieces, in particular by drilling holes; puncturing a biological cell, in particular to deliver a substance to the biological cell; and surface cleaning of workpieces.

In an embodiment, the at least one device for delivering an energy pulse is capable of delivering an energy pulse with an amount of energy at least equal to, in particular at least three times, an amount of energy needed to created a vapour bubble with an initial diameter equal to an inside diameter of a conduit section.

This ensures the creation of a sufficiently large pressure pulse, assuming that the energy pulse is also of a relatively short duration. Suitable pulse durations are in the order of tens of nanoseconds or shorter, e.g. 10 ns or shorter.

In an embodiment, the at least one device for delivering an energy pulse includes at least one device, in particular a pulsed laser, for delivering a pulsed beam of radiation, in particular a pulsed beam of light.

This is a good way of delivering the required high amount of energy in a sufficiently short time. A relatively strong pressure pulse can be delivered to the meniscus. A normal heater is not able to deliver sufficient energy in a pulse of sufficiently short duration, because of the short diffusion length of the heat pulse into the liquid. By heating a much bigger liquid volume impulsively, a bigger pressure pulse is created. This pressure pulse is due to explosive boiling, i.e. rapid vapour formation from a thermodynamically metastable liquid.

In an embodiment, the at least one arrangement for forming a meniscus includes an arrangement, in particular a hydrophobic coating, for repelling liquid from an inside of a wall of the conduit section along at least a part of its length, in particular along only part of its length.

This is a mechanism for forming an at least partly concave meniscus that is easy to implement. In particular where a hydrophobic coating is used, an at least partly concave meniscus forming an interface between a liquid of the same polarity as water and the environment can be formed. Actuators, controllers or a source of energy are not required to form and maintain a meniscus of this shape. The slug forming the liquid jet is easier to control when the hydrophobic coating is provided on the inside of the wall of the conduit section along only part of its length. The curvature of the meniscus, which partly determines the characteristics of at least the first stage of the jet, can be adjustable in certain variants.

An embodiment of the device includes an arrangement, in particular a hydrophobic coating, for repelling liquid from an outside of a wall of each conduit section along at least a part of its length extending to the end open to the environment.

This prevents liquids with the same polarity as the liquid from which the jet is formed, e.g. water, from adhering to the outside of the wall of the conduit section, at least at its opening, prior to the jet emerging. As a consequence, a directed jet is formed, also when a thin first stage of the jet is followed by a subsequent thicker stage of the jet. Premature disruption and defocusing of the jet are prevented.

According to another aspect, the method according to the invention is characterised by causing at least a first stage of a jet to emerge from a central part of the meniscus by delivering an energy pulse creating a pressure pulse, in particular a shock front, propagated through the liquid in the conduit section to the meniscus.

This method is the counterpart to the device according to the invention, providing substantially similar effects.

In an embodiment, the pressure pulse, in particular a shock front, is created by delivering an energy pulse creating a vapour bubble in liquid in a holder in fluid communication with the conduit section.

This embodiment finds many practical applications, because it can be implemented using a relatively compact device that can be held still during use. Due to the extremely thin high-velocity jets that can be generated with the method according to the invention, it is possible to put such applications into effect without causing collateral damage, e.g. bruising in the case of needleless injection, wetting of neighbouring components in the case of localised cooling, destruction of biological cells in the case of delivery of compounds to the cell, etc.

In a variant of this embodiment, the step of delivering an energy pulse to create a vapour bubble includes delivering a pulsed beam of radiation having a peak in its spectrum corresponding to a peak in the absorption spectrum of at least a component of the liquid.

Thus, more energy can be delivered and absorbed by the liquid in a shorter time, increasing the amount of vapour that is created due to flash boiling, and thus providing a stronger and sharper pressure pulse that propagates in the direction of the meniscus.

In an embodiment, the vapour bubble is caused to expand into the conduit section.

Thus, a relatively accurately metered dose of liquid can be delivered with the fluid jet, albeit that not all liquid is necessarily provided with the first stage of the fluid jet. Either the vapour bubble collapses or it vents into the environment, indicating that generally all of the liquid has been ejected from the conduit section.

In an embodiment of the method, a second stage of the jet, having a diameter determined generally by a diameter of an orifice of the conduit section, is expelled from the conduit section.

This embodiment can be used in, for example, administering needleless injections. An effect of this embodiment is to enable a relatively large volume of liquid to be delivered. The first stage of the jet can be used to puncture a surface, e.g. the skin of an animal such as a human being, in a relatively effective way without collateral damage. The liquid of the second stage jet is then delivered through the puncture already made. The fact that the diameter of the second stage is determined by the diameter of an orifice does not imply that it corresponds exactly. There may be a certain amount of constriction or expansion. It is, however, independent of the curvature of the meniscus.

In an embodiment, at least the first stage of the jet has a maximum velocity during its lifetime of at least 100 m/s, in particular at least 300 m/s.

Thus, this embodiment is suitable for such applications as administering needless injections.

According to another aspect of the invention, a device is provided for providing an injection to an organism, including at least one device for creating at least one microfluidic jet according to the invention and/or configured to carry out a method according to the invention.

The organism can be an animal, in particular a human being, or the device can be adapted to injecting liquids into plants or plant material. In contrast to known devices of this type, the device is able to deliver relatively narrow and fast jets of liquid. At least the diameter is smaller than could be achieved by narrowing the conduit section. Maximum jet velocities of up to 640 m/s have been achieved for jets with diameters below 50 µm. Small jet diameters limit the penetration depth of jets, helping to avoid bruising in the case of devices arranged to provide an injection to a human being or other animal. High velocities ensure that the skin is nevertheless penetrated by the jet.

According to another aspect of the invention, there is provided a use of a device for creating at least one microfluidic jet according to the invention and/or of a method according to the invention in any one of the following activities:
administering at least one substance to an animal, in particular a human being;
administering at least one substance to at least part of a plant or fungus;
puncturing a part of an organism;
localised cooling of device components, in particular electrical device components;
machining workpieces, in particular by drilling holes;
puncturing a biological cell, in particular to deliver a compound to the biological cell;
surface cleaning of workpieces.

All these applications can be carried out with more precision using liquid jets of which at least a first stage has a diameter of 100 µm or lower and a velocity of 100 m/s or higher. These can be created by passing a shock front over a curved meniscus forming an interface between liquid in the conduit section and an environment. The administration of at least one substance involves transdermal delivery of the at least one substance without the use of needles.

The invention will be explained in further detail with reference to the accompanying drawings, in which:
Fig. 1 is a schematic block diagram of an embodiment of a device for creating at least one jet of liquid;
Fig. 2 is a schematic diagram of part of such a device;
Figs. 3A-3B show in a schematic way three stages in the formation of a jet of liquid using a device as illustrated in Figs. 1 and 2;
Fig. 4 is a schematic diagram of an experimental set-up used to prove the concept behind the device illustrated in Figs. 1 and 2;
Fig. 5 is a diagram showing the maximum velocities of first and second stages of liquid jets obtained using the experimental set-up illustrated in Fig. 4; and
Fig. 6 is a photograph of a liquid jet obtained using the actual experimental set-up.

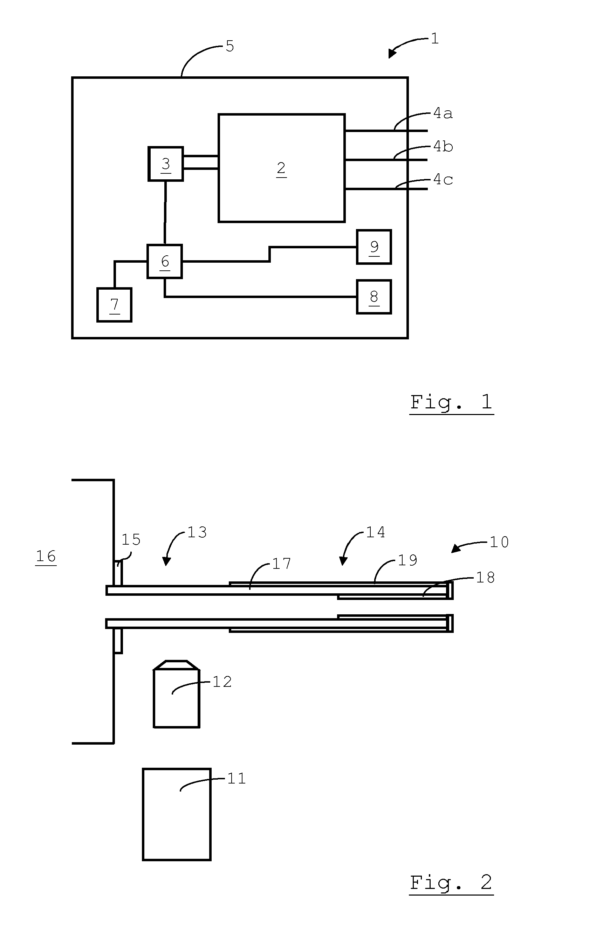

Basic components of a device 1 for creating multiple jets of liquid are shown in Fig. 1. In the illustrated embodiment, the device 1 includes a refillable reservoir 2 containing liquid and a transfer device 3 for transferring liquid into one or more of a plurality of conduits 4a-c, in fluid communication with the reservoir 2. Ends of the conduits 4a-c emerging from a housing 5 of the device 1 are open to the environment. In one variant, the device 3 comprises a plurality of devices for selectively transferring liquid into one or more of the conduits 4a-c.

In an alternative embodiment, the refillable reservoir 2 is dispensed with. Instead, the conduits 4a-c can be dipped into liquid that is sucked up into the conduits 4a-c, either because they are capillaries or because a device (not shown) is provided that lowers the pressure in the conduits 4a-c.

In yet another embodiment, the conduits 4a-c are replaceable. In this embodiment, a user simply mounts them into the device 1 in a pre-filled state, and removes them after use.

The operation of the device 1 is determined by a controller 6, and it is provided with user controls 7 enabling a user to operate the device 1.

When liquid is present in the conduits 4a-c, a meniscus forming an interface between the liquid and the environment to which the conduit is open is or can be created within the conduit at its end open to the environment. Each meniscus is concave, seen looking into the conduit through the end open to the environment.

The device 1 further includes at least one device for delivering an energy pulse that creates a shock wave propagating through at least one of the conduits 4a-c to the concave meniscus in that conduit 4. As a result, a jet is expelled through the end of the conduit 4. At least a first stage of this jet emerges from a central part of the meniscus (i.e. close to a longitudinal axis of the conduit 4 concerned).

A similar jet formation can be found in Antkowiak, A. et al., "Short-term dynamics of a density interface following an impact", J. Fluid Mech., Vol. 577, 2007, pp. 241-250. This describes the generation of a pressure pulse by dropping a column of water on the floor, making it unsuitable for the type of applications for which the devices 1 described herein are configured, in particular for generating jets with a diameter in the order of micrometers.

In the device 1 described herein, the shock wave is created by creating a vapour bubble in liquid with an unimpeded passage to the end of at least one of the conduits 4a-c. To do this, a sufficiently short and powerful energy pulse to create a vapour bubble by explosive boiling is delivered. The device for delivering an energy pulse includes a device 8 for delivering a pulsed beam of radiation and a beam processing device 9 for delivering the pulsed beam of radiation to at least one of the conduits 4a-c. To this end, the conduits 4a-c are transparent to the type of radiation used at at least one location along their length. As illustrated, the beam processing device 9 is controllable by the controller 6, but this need not necessarily be the case.

In certain embodiments, the device 8 for delivering a pulsed beam of radiation is a device for delivering a pulsed beam of optical radiation. In an embodiment that is relatively easy to implement, it is a pulsed laser. In another embodiment, it is a light-emitting diode capable of delivering sufficient energy in a sufficiently short pulse that a shock wave is created. Yet another alternative device 8 includes an X-ray generator. In an embodiment, a liquid is used that includes at least a component having a peak in its absorption spectrum matching the spectrum of radiation emitted by the device 8 for delivering a pulsed beam of radiation, in particular a peak therein. An excipient with such absorption properties can be added to the active ingredient of a composition to be injected into plant or animal, in particular a mammalian animal such as a human being.

In an embodiment, the beam processing device 9 includes a system for focusing the beam from the device 8 for delivering a pulsed beam of radiation to a spot in a selected one of the conduits 4a-c. This can include one or more optical switches, for example. The beam processing device 9 can also be a focusing device, allowing the conduits 4a-c to be used one after the other.

In an alternative embodiment, the beam processing device 9 is dispensed with, so that jets can emerge from all conduits 4a-c filled with liquid at the same time. To this end, the pulsed beam of radiation is passed laterally through the conduits 4a-c.

In another embodiment, (not illustrated in detail), the pulsed beam of radiation enters a conduit 4 along its longitudinal axis, and is focused to a spot at a certain distance from the end open to the environment.

In yet another embodiment, the pulsed beam of radiation, e.g. the laser beam, is not delivered to a spot within a conduit 4, but is delivered to a holder of liquid that is in fluid communication with several of the conduits 4a-c, e.g. the reservoir 2. This allows for multiple jets to be created at substantially the same time. The device 8 for providing an energy pulse will be arranged to provide a correspondingly more powerful energy pulse. In this embodiment, the vapour bubble is caused to expand into each of the conduits 4a-c before collapsing. However, it is noted that creating parallel jets by creating separate vapour bubbles in each of the conduits 4a-c allows for the delivery of a jet with a more precisely defined volume.

A detailed view of part of a device for creating a jet of liquid having a single conduit 10 is given in Fig. 2. The following description is however equally applicable to the conduits 4a-c of the device 1 of Fig. 1. Fig. 2 shows a laser, in particular a pulsed laser 8, and an objective 12 for focusing a beam from the pulsed laser 11 to a spot within a first section 13 of the conduit 10. The first section 13 is located adjacent a second section 14 that is open to the environment at one end. A mounting device 15 is provided for holding the conduit 10. In the illustrated embodiment, the conduit 10 is open to a reservoir 16 at an end opposite the end open to the environment. In an alternative embodiment, the end opposite to the end open to the environment is closed.

The conduit 10 in the illustrated embodiment is a capillary made of glass. In other embodiments, the conduit 10 is made of a different material. Although the conduit 10 will generally be circle-cylindrical, it can have a non-circular cross-section, e.g. rectangular or quadratic. In the illustrated embodiment, the conduit 10 has an inside diameter below 200 µm, more particularly below 100 µm.

As mentioned in connection with the device 1 of Fig. 1, an arrangement is provided for forming the meniscus. In principle, this can be a device for establishing a pressure level in the opening of the conduit 10 that results in the formation of a meniscus that is at least partially concave (i.e. has a dimple in at least the middle). Easier is an arrangement for repelling liquid of at least a certain type. In one embodiment (not shown), electrowetting is used to change the contact angle of the edges of the meniscus to the inside of a wall 17 of the conduit 10. Thus, the arrangement for forming the meniscus includes a device for establishing a variable electrical field. Such a device can operate under the control of the controller 6, so that the contact angle of the meniscus to the inside of the wall of the conduit 10 can be set to an appropriate value. This is useful in that it provides a way of adapting the diameter of at least the first stage of the jet independently of its velocity.

In the illustrated embodiment, an inside of the wall 17 forming a boundary to the second section 14 is provided with a hydrophobic coating 18 along only part of the length of the second section 14 up to the end open to the environment. This coating 18 can be a silane, for example, or Teflon or FDTS (1H-,1H-,2H-,2H-perfluorodecyltrichlorosilane). It may also be a material that is not inherently hydrophobic, but made superhydrophobic by virtue of a particular nanoscale structure, for example carbon nanofibre jungles.

In this embodiment, the curvature of the meniscus, and thus the diameter of at least the first stage of the jet, can be varied by adapting the composition of the liquid.

The wall 17 of the conduit 10 is also coated on the outside with a hydrophobic coating 19 along at least part of its length up to the end of the conduit 10 open to the environment. This ensures that no liquid can adhere to the outside of the conduit 10 at the end where the jet emerges. In particular, the jet will not strike or mix with liquid adhered to that end. This drastically reduces the risk of splattering and break-up of the jet.

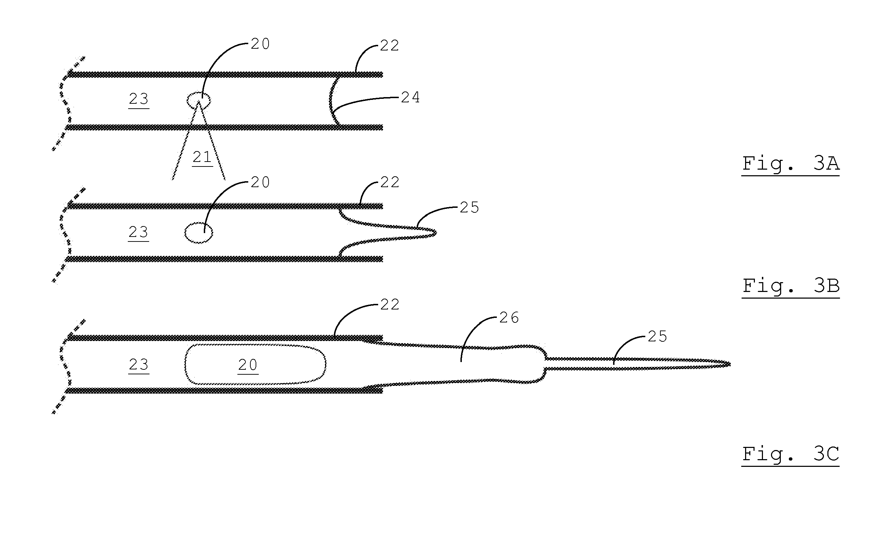

Stages in the formation of a jet are shown in Figs. 3A-3B. A vapour bubble 20 is created by means of a pulsed, in particular focused, laser beam 21 in a conduit 22 filled with liquid 23 and provided with an arrangement for forming a meniscus 24 that is at least partially concave, seen looking into the conduit section through the end open to the environment. The result is a pressure impulse that propagates towards the meniscus, as a result of which a thin jet 25 is formed. The thin jet 25 emerges from a central part of the meniscus 24 (Fig. 3B). It will be apparent that the diameter of the thin jet 25 is lower than a minimum inside diameter of the conduit 22. The diameter of the thin jet depends on the curvature of the meniscus 24, the size of the pressure impulse, and its gradient with respect to time.

As the vapour bubble 20 expands further (Fig. 3C), the first stage of the jet (the thin jet 25) is followed by a second stage in the form of a thick jet 26, having a diameter depending on an inside diameter of the conduit 22. The thin jet 25, which has a very high velocity and low diameter, is able to penetrate many surfaces, e.g. the skin of a human being or non-human mammal or the membrane or wall of a cell, whereas the thick jet 26 carries a larger volume of liquid. The vapour bubble 20 is caused to expand to a stage in which it reaches the end of the conduit 22 open to the environment, whereupon it ruptures. At that stage, essentially all of the liquid between the spot where the vapour bubble 20 was created and the meniscus 24 has been expelled. In an alternative embodiment, the bubble collapses without venting.

The properties of the laser pulse that is used to create the vapour bubble 20 are determined as follows. Firstly, the pulse duration must be shorter than the time scale of the thin jet 25. The time scale can be estimated as the ratio of the inside diameter of the conduit 22 to the required velocity of the thin jet 25. For a typical jet length of 100-500 µm and a maximum velocity of 300 m/s, this implies a pulse duration that is much shorter than 500 ns, preferably at least one order of magnitude shorter.

Moreover, the energy in the pulse must be enough to vaporise the required amount of liquid. One can assume a maximum bubble length L and diameter D, with D equal to the inside diameter of the conduit 22. An approximation of the length L is the distance between the point at which the pulsed laser beam 21 is focused and the location of the apex of the curved meniscus along the longitudinal axis of the conduit 22. The maximum volume of the bubble 20 is equal to π(*^{D}*/₂)²·*L*. Assuming that the mass of the vapour bubble is equal to the mass of liquid from which it was created, the latter can be calculated as ρ*ᵥₐₚₒᵤᵣ*·π(*^{D}*/₂)²·*L*, where a typical value for ρ*ᵥₐₚₒᵤᵣ* is 2.6 kg·m⁻³ for saturated water at 150°C. The energy required to vaporise this amount of liquid is *E*≈*Hₗₐₜₑₙₜ* · ρ*ᵥₐₚₒᵤᵣ* ·*π(^{D}*/*₂)*². For the example given here, this would amount to about 5 µJ. Experiments suggest that the laser pulse should have a few times this amount of energy, because the absorption of the laser energy is not perfect.

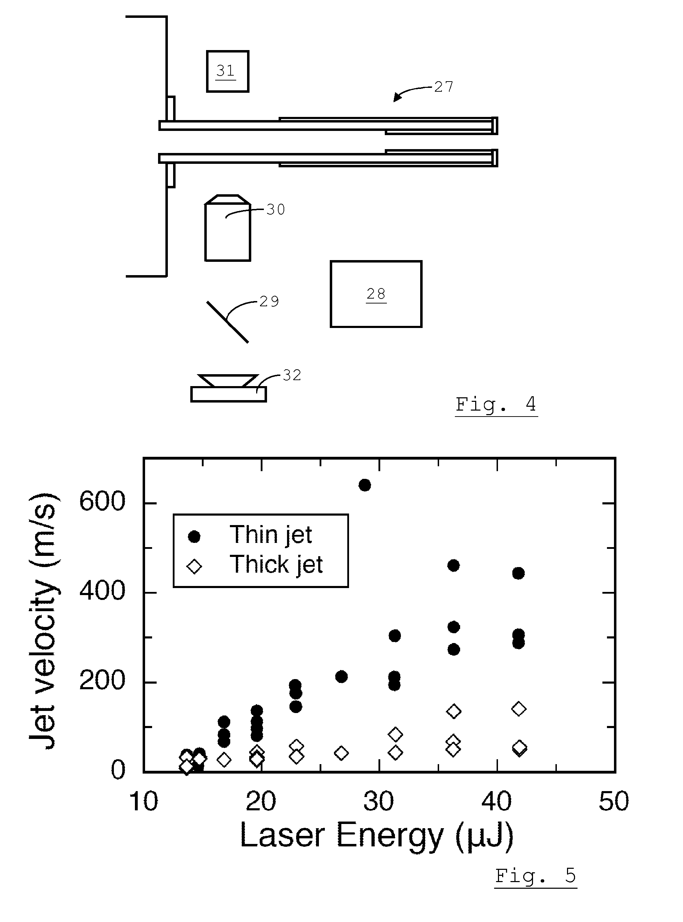

A prototype device for creating a jet of liquid has been built, and is illustrated schematically in Fig. 4. There is shown a conduit 27 having essentially the same construction as the conduit 22 shown in Fig. 3. In experiments carried out with the prototype device, a pulsed laser beam from a laser 28 was directed by a dichroic filter 29 through an objective 30 to a spot in the conduit 27. A strobed light-emitting diode 31 and camera 32 were used to capture images of the jet.

The prototype device is representative for all the devices discussed above. The conduit 27 is made of glass, and has an outer diameter of 80 µm and an inner diameter of 50 µm. Its length is 20 mm. The laser 28 is a Nd:YAG laser providing light pulses with a wavelength of 532 nm and pulse duration of 6 ns.

Fig. 5 is a diagram showing the maximum velocity of the thin and the thick jet against the energy in the laser pulse. It will be apparent that velocities of up to 640 m/s for the first stage of the jet were achieved. The second, thicker stage had maximum velocities in the region of 140 m/s.

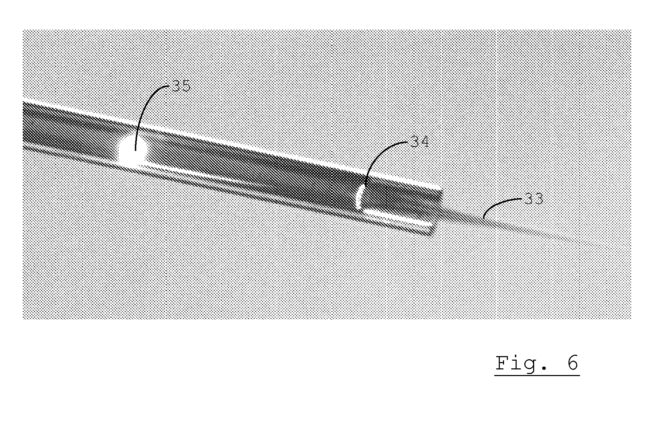

A representative image of a jet is shown in Fig. 6. The brightness of this image has been enhanced to allow for better reproduction and visibility, but the image has not otherwise been post-processed. One can see the first stage of the jet (a thin jet 33) and a remaining outer edge of a meniscus 34, as well as a vapour bubble. Due to the speed and diameter, this jet is suited very well to use in administering needleless injections. A larger dose can be delivered using the device 1 of Fig. 1.

Other applications of the devices outlined above are, however, also conceivable.

One application is in the delivery of substances to at least part of a plant, i.e. to a plant or to plant material. For example, it would be possible to deliver a substance to a seed with little risk of creating a large hole in the seed coat or even destroying the seed.

Another application is in puncturing part of an organism, e.g. an animal, plant or fungus. This can be biological material obtainable from a living animal, including a human being, or a plant seed, for example to induce germination. The devices outlined herein are in some embodiments suitable for puncturing part of a living animal, including a human being, e.g. to carry out an operation.

A related application is in the delivery of DNA or RNA to plant or animal (including human) cells. Due to the small diameter of the thin jet 25, a small enough hole can be made in the cell membrane or cell wall that the cell is not destroyed. Of course, other compounds or compositions can be delivered to the cell, such as markers for imaging purposes.

Another application is in the localised cooling of devices, notably electronic devices such as integrated circuits. The jet can, for example, be directed onto a very small heat sink, but not the rest of the device, or only onto such components as are not at risk of being damaged by liquid jets.

Another application is in the micro-machining of workpieces, in particular by drilling holes. These can be very small workpieces, e.g. microfluidic devices, sieves, optical gratings, etc. In this regard, the term "machining" is used to denote all types of processing of workpieces in which the identity of the workpiece is changed by (locally) shaping the material thereof. For example, coatings can be removed locally, or an inscription can be made in the workpiece. Such inscriptions are useful for marking products for anti-counterfeiting purposes, for example.

Another application of the jets generated by the device disclosed herein is in the surface cleaning of workpieces. This can in particular include locally removing parts of a mask or other coating of a workpiece, e.g. in lithographic processes. Larger jets can be used to clean objects made of precious metals (e.g. jewellery) or of glass, for example, where it is desirable to remove as little of the material of the object to be cleaned as possible.

All these applications have in common that they benefit from the use of high-velocity microjets with very small diameters, such as those created using the devices described above.

The invention is not limited to the embodiments described above, which can be varied within the scope of the accompanying claims. This scope can be limited to exclude all such uses of the methods outlined herein insofar as they include a method of treatment of the human or animal body by surgery, meaning that such methods that comprise or encompass an invasive step representing a substantial physical intervention on the body which requires professional medical expertise to be carried out and which entail a substantial health risk even when carried out with the required professional care and expertise are excluded.

The elements described herein can individually be essential to the invention in all its various forms or in any combination. For example, where mention is made of a liquid herein, this includes mixtures and suspensions. Although the conduit section in which the meniscus is formed is approximately circle-cylindrical in the illustrated embodiments, it may taper or include a convergent-divergent nozzle, for example. However, because the diameter of at least the first stage of the jet is not determined by the diameter of the conduit, a simple circle-cylindrical conduit is adequate. The liquid in the holder in which a vapour bubble is created need not be the same as that used to create the jet. Two immiscible liquids can be used, with an appropriately higher energy pulse being delivered.

### LIST OF REFERENCE NUMERALS

1 - Device for creating a liquid jet
2 - Reservoir
3 - Transfer device
4a-c - Conduits
5 - Housing
6 - Controller
7 - User controls
8 - Device for delivering a pulsed beam of radiation
9 - Beam processing device
10 - Conduit
11 - Laser
12 - Objective
13 - First conduit section
14 - Second conduit section
15 - Mounting device
16 - Reservoir
17 - Wall
18 - Coating on inside of conduit
19 - Coating on outside of conduit
20 - Vapour bubble
21 - Beam
22 - Conduit
23 - Liquid
24 - Meniscus
25 - Thin jet
26 - Thick jet
27 - Conduit
28 - Laser
29 - Dichroic filter
30 - Objective
31 - Strobed light-emitting diode
32 - Camera
33 - Thin jet
34 - Meniscus
35 - Vapour bubble

## Claims

1. Device for creating at least one microfluidic jet, including:
at least one conduit section (4a-c;14;22;27), open to an environment of the device at one end thereof;
at least one arrangement (18) for forming in at least one of the conduit sections (4a-c;14;22;27) a meniscus (24;34) forming an interface between liquid (23) in the conduit section (4a-c;14;22;27) and the environment, at a position within the conduit section (4a-c;14;22;27),
wherein the meniscus (24;34) is at least partially concave, seen looking into the conduit section (4a-c;14;22;27) through the end open to the environment; and
at least one holder (13) for holding liquid (23), in fluid communication with at least one of the conduit sections (4a-c;14;22;27), **characterised by**
at least one device (8;11;28) for delivering an energy pulse to liquid (23) in at least one of the holders (13), configured to create a pressure pulse, in particular a shock front, propagating to the meniscus (24;34) and causing at least a first stage (25;33) of a jet to emerge from a central part of the meniscus (24;34).

2. Device according to claim 1, wherein the at least one device (8;11;28) for delivering an energy pulse is configured to create the pressure pulse, in particular a shock front, by creating a vapour bubble (20;35) in liquid (23) in the at least one holder (13).

3. Device according to claim 2, wherein the at least one device (8;11;28) for delivering an energy pulse is capable of delivering an energy pulse with an amount of energy at least equal to, in particular at least three times, an amount of energy needed to created a vapour bubble (20;35) with an initial diameter equal to an inside diameter of a conduit section (4a-c;14;22;27).

4. Device according to any one of the preceding claims, wherein the at least one device (8;11;28) for delivering an energy pulse includes at least one device (8;11;28), in particular a pulsed laser, for delivering a pulsed beam of radiation, in particular a pulsed beam of light.

5. Device according to any one of the preceding claims, wherein the at least one arrangement (18) for forming a meniscus includes an arrangement, in particular a hydrophobic coating, for repelling liquid from an inside of a wall (17) of the conduit section (4a-c;14;22;27) along at least a part of its length, in particular along only part of its length.

6. Device according to any one of the preceding claims, including an arrangement (19), in particular a hydrophobic coating, for repelling liquid (23) from an outside of a wall (17) of each conduit section (4a-c;14;22;27) along at least a part of its length extending to the end open to the environment.

7. Method of creating at least one microfluidic jet, including:
providing at least one conduit section (4a-c;14;22;27), open to an environment at one end; and
providing liquid (23) within at least one of the conduit sections (4a-c;14;22;27) up to a meniscus (24;34) forming an interface between liquid (23) in the conduit section (4a-c;14;22;27) and the environment,
wherein the meniscus (24;34) is situated at a position within the conduit section (4a-c;14;22;27), and
wherein the meniscus (24;34) is at least partly concave, seen looking into the conduit section (4a-c;14;22;27) through the end open to the environment, **characterised by**
causing at least a first stage (25;33) of a jet to emerge from a central part of the meniscus (24;34) by delivering an energy pulse creating a pressure pulse, in particular a shock front, propagated through the liquid (23) in the conduit section (4a-c;14;22;27) to the meniscus (24;34).

8. Method according to claim 7, wherein the pressure pulse, in particular a shock front, is created by delivering an energy pulse creating a vapour bubble (20;35) in liquid (23) in a holder (13) in fluid communication with the conduit section (4a-c;14;22;27).

9. Method according to claim 8, wherein the step of delivering an energy pulse to create a vapour bubble (20;35) includes delivering a pulsed beam of radiation having a spectrum matching a peak in the absorption spectrum of at least a component of the liquid (23).

10. Method according to claim 8 or 9, wherein the vapour bubble (20;35) is caused to expand into the conduit section (4a-c;14;22;27).

11. Method according to any one of claims 7-10, wherein a second stage (26) of the jet, having a diameter determined generally by a diameter of an orifice of the conduit section (4a-c;14;22;27), is expelled from the conduit section (4a-c;14;22;27).

12. Method according to any one of claims 7-11, wherein at least the first stage (25;33) of the jet has a maximum velocity during its lifetime of at least 100 m/s, in particular at least 300 m/s.

13. Device for providing an injection to an organism, including at least one device according to any one of claims 1-6 and/or configured to carry out a method according to any one of claims 7-12.

14. Use of a device according to any one of claims 1-6 and/or of a method according to any one of claims 7-12 in any one of the following activities:
administering at least one substance to an animal, in particular a human being;
administering at least one substance to at least part of a plant or fungus;
puncturing a part of an organism;
localised cooling of device components, in particular electrical or electronic device components;
machining workpieces, in particular by drilling holes;
puncturing a biological cell, in particular to deliver a compound to the biological cell;
surface cleaning of workpieces.
